# EUROPÄISCHE PATENTSCHRIFT

(11) **EP 2 307 340 B1**
(45) Veröffentlichungstag und Bekanntmachung des Hinweises auf die Patenterteilung: **14.08.2013**
(21) Anmeldenummer: 09776832.9
(22) Anmeldetag: 25.06.2009
(51) Int. Cl.: C07C 29/132, C07C 29/149, C07C 31/20

(54) **VERFAHREN ZUR GEWINNUNG VON NEOPENTYLGLYKOL DURCH SPALTUNG VON IM HERSTELLVERFAHREN ANFALLENDEN HOCHSIEDERN**
PROCESS FOR OBTAINING NEOPENTYL GLYCOL BY CRACKING HIGH BOILERS OCCURRING IN THE PRODUCTION PROCESS
PROCÉDÉ DE PRODUCTION DE NÉOPENTYLGLYCOL PAR SCISSION DE COMPOSÉS À HAUT POINT D'ÉBULLITION ISSUS DU PROCESSUS DE PRODUCTION

(30) Priorität: 15.07.2008 DE 102008033163
(43) Veröffentlichungstag der Anmeldung: 13.04.2011
(73) Patentinhaber: OXEA GmbH, 46147 Oberhausen (DE)
(72) Erfinder: SCHULZ, Rolf-Peter, 65830 Kriftel (DE); SCHOLZ, Horst, 46535 Dinslaken (DE); HUPPERICH, Thomas, 46514 Schermbeck (DE); HEYMANNS, Peter, 45147 Essen (DE); WEBER, Tonia, 64293 Darmstadt (DE); KAUFMANN, Alexander, 46539 Dinslaken (DE); SCHALAPSKI, Kurt, 46147 Oberhausen (DE); STRUTZ, Heinz, 47445 Moers (DE)
(86) Internationale Anmeldenummer: PCT/EP2009/004575
(87) Internationale Veröffentlichungsnummer: WO 2010/006688

(56) Entgegenhaltungen:
- EP-A1- 0 006 460
- DE-A1- 1 518 784
- DE-A1- 19 948 112
- DE-B- 1 269 605
- US-A1- 2008 167 506

## Beschreibung

Die vorliegende Erfindung betrifft ein Verfahren zur Gewinnung von Neopentylglykol durch hydrierende Spaltung von im Herstellverfahren anfallenden Hochsiedern in Gegenwart von Kupfer-Chromit-Katalysatoren.

Mehrwertige Alkohole oder Polyole besitzen als Kondensationskomponente zum Aufbau von Polyestern oder Polyurethanen, Kunstharzlacken, Schmiermitteln und Weichmachern eine erhebliche wirtschaftliche Bedeutung. Hierbei sind besonders solche mehrwertigen Alkohole von Interesse, die durch eine gemischte Aldolkondensation von Formaldehyd mit iso- oder n-Butyraldehyd erhalten werden. Bei der Aldolkondensation zwischen Formaldehyd und dem entsprechenden Butyraldehyd bildet sich zunächst eine aldehydische Zwischenstufe, die anschließend zum mehrwertigen Alkohol reduziert werden muss. Ein technisch bedeutender, mehrwertiger Alkohol, der nach diesem Verfahren zugänglich ist, ist Neopentylglykol [NPG, 2,2-Dimethylpropandiol-(1,3)] aus der Mischaldolisierung von Formaldehyd und iso-Butyraldehyd.

Die Aldolreaktion wird mit äquimolaren Mengen in Gegenwart basischer Katalysatoren, beispielsweise Alkalihydroxiden oder aliphatischen Aminen durchgeführt und liefert zunächst das isolierbare Zwischenprodukt Hydroxypivalinaldehyd (HPA). Dieses Zwischenprodukt kann anschließend gemäß DE 1 800 506 A1 mit überschüssigem Formaldehyd entsprechend der Cannizzaro-Reaktion in Neopentylglykol unter Bildung eines Äquivalents eines Formiatsalzes überführt werden. Technisch umgesetzt wird aber auch die katalytische Hydrierung von Hydroxypivalinaldehyd in der Gas- und Flüssigphase am Metallkontakt. Als geeignete Hydrierkatalysatoren haben sich gemäß EP 0 278 106 A1 Nickelkatalysatoren erwiesen. Katalysatoren auf Basis von Kupfer, Zink und Zirkonium werden in dem Verfahren nach EP 0 484 800 A2 in der Hydrierstufe eingesetzt. Nach der Lehre aus EP 0 522 368 A1 kann der Hydrierschritt mit Kupfer-Chromit-Katalysatoren unter besonders schonenden Druck- und Temperaturbedingungen durchgeführt werden. Gemäß US 4,855,515 A1 zeichnen sich besonders Mangan dotierte Kupfer-Chromit-Katalysatoren als Hydrierkatalysatoren bei der Hydrierung des Adolisierungsproduktes aus der Umsetzung von Formaldehyd mit iso-Butyraldehyd aus.

Der nach EP 0 522 368 A1 bekannte Hydrierprozess wird in Gegenwart von mindestens 20 Gew.-%, eines niederen Alkohols, durchgeführt, wobei sich die Mengenangabe auf das Gemisch aus Alkohol und Aldolisierungsprodukt bezieht. Dieser Stand der Technik nimmt ebenfalls für sich in Anspruch, dass nicht nur Hydroxypivalinaldehyd sondern auch dass sein Disproportionierungsprodukt nach der Tischtschenko-Reaktion, 2,2-Dimethyl-1,3-propandiol-monohydroxypivalinsäureester (HPN), unter den Reaktionsbedingungen in Neopentylglykol gespalten wird.

Unabhängig davon, ob Neopentylglykol nach dem Cannizzaro- oder nach dem Hydrierverfahren hergestellt wird, fallen in der Aldolisierungsstufe eine Reihe von Nebenkomponenten an, die als Derivate des Neopentylglykols angesehen werden können. Aufgrund der hohen Reaktivität des Formaldehyds und des iso-Butyraldehyds sowie aufgrund der hohen Funktionalität der gebildeten Produkte, wie Hydroxyaldehyde und mehrwertige Alkohole, können eine Reihe von Nebenreaktionen, wie die Tischtschenko-Reaktion, Acetalisierung oder Esterbildung ablaufen, die zur Bildung von Hochsiedern führt.

Im Falle der Umsetzung von Formaldehyd mit iso-Butyraldehyd handelt es sich bei den Hochsiedern unter anderem um folgende Verbindungen:

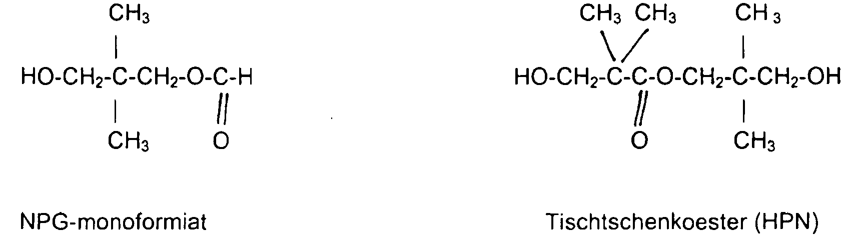

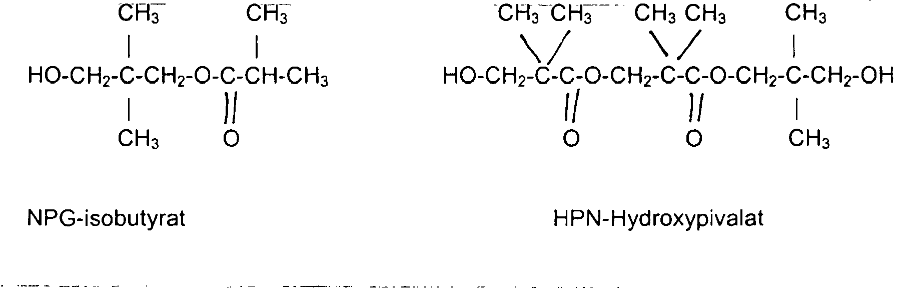

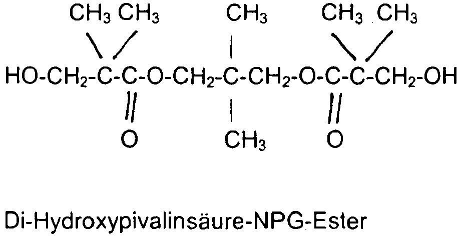

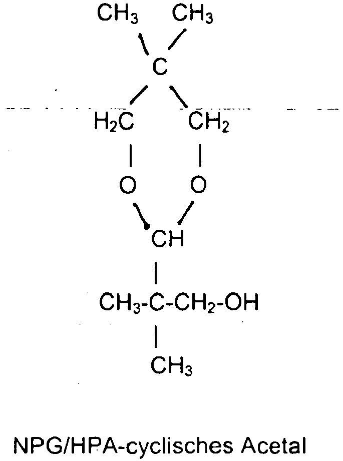

In den Hochsiedern aus der Neopentylglykolherstellung finden sich somit sauerstoffhaltige Verbindungen wie höhersiedende Ester oder Acetale.

Die Hochsieder können an verschiedenen Stellen aus dem Herstellprozess ausgeschleust werden, beispielsweise nach dem Aldolisierungsschritt als Sumpfprodukt aus einem vor der Hydrierstufe vorgeschalteten Verdampfer, wobei das am Kopf des Verdampfers abgezogene rohe Aldolisierungsprodukt in der Gasphase am Metallkotakt mit Wasserstoff zum Neopentylglykol hydriert wird. Auch bei der destillativen Reinigung zu dem gewünschten Neopentylglykol fallen Hochsieder an, beispielsweise als Sumpfprodukt in der abschließenden Reindestillationskolonne.

Dieser Hochsiederanfall ist unerwünscht, da in ihm Einheiten des mehrwertigen Alkohols chemisch gebunden sind und die Ausbeute an dem gewünschten Neopentylglykol deutlich erniedrigt wird. Ebenfalls sind in dem Hochsieder auch noch erhebliche Mengen an Neopentylglykol physikalisch eingemischt. Ferner können Spuren von Hochsiedern im Endprodukt auch die Anwendungseigenschaften negativ beeinflussen.

Aus WO97/01523 A1 ist bekannt, die bei der Herstellung von Neopentylglykol durch Reaktion mit Formaldehyd anfallenden cyclischen Acetale in wässriger saurer Lösung oder Suspension in Gegenwart von Metallkatalysatoren mit Wasserstoff bei erhöhter Temperatur und bei erhöhtem Druck in die entsprechenden Diole und in Formaldehyd zu spalten. Der freigesetzte Formaldehyd wird unter den Reaktionsbedingungen zu Methanol hydriert.

Nach der Lehre von DE 1 518 784 A1 werden bei der Herstellung von Neopentylglykol die bei der Reinigung des Hydrierprodukts in der abschließenden Reindestillationskolonne anfallenden Hochsieder zumindest teilweise in den Hydrierreaktor zurückgeführt, wobei nach der bekannten Verfahrensweise das zu hydrierende Aldolisierungsrohprodukt einen Überschuss an iso-Butyraldehyd enthält. Die katalytische Hydrierung des Aldolisierungsrohproduktes erfolgt in Gegenwart von Kupfer-Chromit-Katalysatoren.

EP 0 006 460 A1 offenbart ein Verfahren zur Herstellung von reinem Neopentylglykol aus rohem Hydroxypivalinaldehyd, der unter anderem monoiso-Buttersäureester des Neopentylglykols enthält. Nach dem bekannten Verfahren wird der rohe Hydroxypivalinaldehyd in zwei Stufen in Gegenwart eines mit Barium aktivierten Kupferchromitkatalysators hydriert.

Aus US 2008/0167506 A1 ist bekannt, iso-Butyraldehyd mit Formaldehyd in Gegenwart eines tertiären Amins zum Hydroxypivalinaldehyd umzusetzen, der anschließend in Gegenwart eines Kupferkatalysators, gegebenenfalls enthaltend Chrom, hydriert wird. Der bekannte Prozess kann mit oder ohne eines organischen Lösungsmittels durchgeführt werden.

Die bekannten Verfahren zur Gewinnung von Neopentylglykol aus den bei ihrer Herstellung anfallenden Hochsiedern erfordert entweder eine Säurebehandlung und den Wasserzusatz mit nachfolgender Hydrierung oder die Anwesenheit eines Lösungsmittels, wenn diese Hochsieder in die Hydrierstufe, in der gleichzeitig auch das zunächst erhaltene Aldolisierungsrohprodukt hydriert wird, zurückgeführt werden.

Überraschenderweise wurde gefunden, dass Neopentylglykol aus den bei ihrer Herstellung anfallenden Hochsiedern auf einfache Weise in hohen Mengen zurückgewonnen werden kann, wenn diese Hochsieder in einem separaten Hydrierreaktor lösungsmittelfrei in Gegenwart von Kupfer-Chromit-Katalysatoren mit Wasserstoff behandelt werden und die erhaltenen Spaltprodukle destillativ aufgearbeitet werden.

Die vorliegende Erfindung betrifft daher ein Verfahren zur Gewinnung von Neopentylglykol aus den bei der Umsetzung von Formaldehyd mit iso-Butyraldehyd anfallenden Hochsiedern. Es ist dadurch gekennzeichnet, dass die Hochsieder aus dem Verfahren zur Herstellung von Neopentylglykol abgetrennt werden und in einem separaten Hydrierreaktor lösungsmittelfrei in Gegenwart eines Kupfer-Chromit-Katalysators bei einer Temperatur von 140 bis 200°C und bei einem Druck von 7 bis 28 MPa mit Wasserstoff in der flüssigen Phase behandelt werden und die erhaltenen Spaltprodukte destillativ aufgearbeitet werden.

Im Gegensatz zu den bekannten Verfahren, bei denen die Hochsieder in die Hydrierstufe zurückgefahren werden, in der gleichzeitig auch das Aldolisierungsrohprodukt aus Formaldehyd mit iso-Butyraldehyd hydriert wird, findet nach dem erfindungsgemäßen Verfahren die Behandlung mit Wasserstoff, die man auch als hydrierende Spaltung der Hochsieder auffassen kann, in einem separaten Hydrierreaktor statt. Durch diese entkoppelte Verfahrensausgestaltung können die Reaktionsbedingungen für die hydrierende Spaltung der Hochsieder gezielt und unabhängig von den in der Hydrierung des Aldolisierungsrohprodukts zu wählenden Reaktionsbedingungen eingestellt werden. Ferner ermöglicht die Durchführung der hydrierenden Spaltung in einem separaten Hydrierreaktor auch die Aufarbeitung von Hochsiedern, die bei der Herstellung und Reinigung von Neopentylglykol nach dem Cannizzaro-Verfahren anfallen, bei dem das Aldolisierungsprodukt mit einem weiteren Äquivalent Formaldehyd umgesetzt wird und nicht mit Wasserstoff hydriert wird.

Die hydrierende Spaltung der Hochsieder erfolgt lösungsmittelfrei. Der Begriff lösungsmittelfrei im Sinne der vorliegenden Erfindung bedeutet, dass bei der Behandlung mit Wasserstoff weder ein organisches Lösungsmittel noch Wasser zugesetzt werden. Allerdings können geringe Mengen an leichtsiedenden Verbindungen, beispielsweise iso-Butanol oder n-Butanol oder Wasser, die Lösungsmitteleigenschaften aufweisen und die in vorgeschalteten Prozess-Schritten zugesetzt oder gebildet werden, im Hochsieder enthalten sein.

Die bei der Herstellung von Neopentylglykol (NPG) anfallenden Hochsieder enthalten die zuvor genannten Sauerstoff haltigen Verbindungen wie NPGmonoformiat, NPG-isobutyrat, HPA-Tischtschenkoester, NPG/HPA-cyclisches Acetal, Di-isobuttersäure-NPG-Ester und Di- hydroxypivalinsäure-NPG-Ester sowie weitere Ester, Ether und Acetalverbindungen. Besonders hoch ist der Gehalt an HPA-Tischtschenkoester und an Di-hydroxypivalinsäure-NPG-Ester. Ferner enthalten die Hochsieder auch noch substantielle Mengen an Neopentylglykol und an Hydroxypivalinaldehyd.

Die für die hydrierende Spaltung einzusetzenden Hochsieder können an den verschiedenen Prozess-Stufen aus dem Verfahren zur Herstellung von Neopentylglykol abgetrennt werden. Sie können dann getrennt oder auch vereinigt zur Gewinnung des gewünschten mehrwertigen Alkohols in die separat durchzuführende hydrierende Spaltung gegeben werden. Bei der Herstellung von Neopentylglykol fallen beispielsweise Hochsieder als Sumpfabzug in einem Verdampfer an, der der Hydnerstufe des Aldolisierungsrohproduktes vorgeschaltet ist. Weitere Hochsieder können bei der destillativen Reinigung des nach Hydrierung anfallenden, rohen Neopentylglykols zur gereinigten Ware ausgeschleust werden. Die vereinigten Hochsiederströme aus der Neopentylglykolherstellung können dann nach dem erfindungsgemäßen Verfahren hydriert werden.

Die hydrierende Spaltung gemäß dem erfindungsgemäßen Verfahren erfolgt in Gegenwart handelsüblicher Kupfer-Chromit-Katalysatoren. Ihre Eignung für die hydrierende Spaltung von Esterverbindungen ist aus dem allgemeinen Stand der Technik bekannt (Ullmann's Encyclopedia of Industrial Chemistry, 5. Auflage, Bd. 9, VCH-Verlag). Kupfer-Chromit-Katalysatoren lassen sich nach H. Adkin, Org. React. 8, 1954, 1-27 als äquimolare Kombination von Kupferoxid und Kupferchromit beschreiben, obgleich sie nicht unbedingt Kupferchromit enthalten. Derartige Hydrierkatalysatoren werden beispielsweise in der DE 26 11 374 A1, DE 1 518 784 A1, US 4,855,515 A1 oder EP 0 522 368 A1 beschrieben. Die Kupfer-Chromit-Katalysatoren enthalten häufig noch Aktivatoren, wie beispielsweise Barium, Cadmium, Magnesium, Mangan und/oder ein seltenes Erdmetall. Die für das Verfahren der Erfindung geeigneten Kupfer-Chromit-Katalysatoren können entweder ohne Trägerstoffe oder mit Trägerstoffen, beispielsweise mit Kieselguhr, Kieselgel oder Aluminiumoxid als Pulver oder in Form von Tabletten, Sternen, Strängen, Ringen oder anderen Teilchen von verhältnismäßig großer Oberfläche verwendet werden.

Besonders geeignet für die erfindungsgemäße Behandlung mit Wasserstoff sind Mangan dotierte Kupfer-Chromit-Katalysatoren.

Die Behandlung mit Wasserstoff oder die hydrierende Spaltung der Hochsieder erfolgt bei Temperaturen von 140 bis 200°C, vorzugsweise von 160 bis 200°C und bei Drücken von 7 bis 28, vorzugsweise von 7 bis 20 MPa ohne Zusatz eines Lösungsmittels und ohne Zusatz von Wasser. Die Anwendung höherer Temperaturen ist nicht empfehlenswert, da eine unselektive Spaltung der mehrwertigen Alkohole in verstärktem Maße einsetzt. Höhere Drücke wirken sich zwar positiv auf die Hydrierleistung des Kupfer-Chromit-Katalysators bei hoher Selektivität zu dem gewünschten mehrwertigen Alkohol aus, doch erfordert das Arbeiten bei hohem Druck auch einen hohen Energieeinsatz, um das Reaktionsgut auf einen entsprechend hohen Druck zu komprimieren.

Die erfindungsgemäße Behandlung mit Wasserstoff wird an fest angeordneten Katalysatoren in der Flüssigphase kontinuierlich oder diskontinuierlich durchgeführt, beispielsweise in einer Riesel- oder Sumpfhydrierung. Daneben kann die Behandlung mit Wasserstoff auch mit suspendierten Katalysatoren erfolgen. Bei der kontinuierlichen Fahrweise hat sich eine Katalysatorbelastung V/Vh, ausgedrückt in Durchsatzvolumen pro Katalysatorvolumen und Zeit, von 0,2 bis 1,2 h⁻¹, vorzugsweise von 0,4 bis 1 h⁻¹ als zweckmäßig erwiesen.

Bei diskontinuierlicher Verfahrensführung verwendet man, bezogen auf das lösungsmittelfreie Einsatzprodukt, 1 bis 10, vorzugsweise 2 bis 6 Gew.-% der vorstehend beschriebenen Kupfer-Chromit-Katalysatoren.

Die aus dem Hydrierreaktor abgezogenen Spaltprodukte werden zunächst in einen Hochdruckabscheider geleitet und auf Normaldruck entspannt. Anschließend werden die Spaltprodukte nach bekannten Destillationsverfahren, beispielsweise gemäß der aus EP 0 278 106 A1 bekannten Arbeitsweise, zu gereinigtem Neopentylglykol aufgearbeitet. In einer besonderen Ausgestaltung werden die Spaltprodukte zunächst mit rohem Neopentylglykol vermischt und anschließend wird die Mischung destillativ aufgearbeitet.

Aus den eingesetzten Hochsiedern kann durch die erfindungsgemäße Behandlung mit Wassertoff Neopentylglikol mit einer Ausbeute von mehr als 80 Gew.-%, bezogen auf Hochsiedereinsatz, zurückgewonnen werden. Das neue Verfahren wird durch die folgenden Beispiele näher erläutert, es ist jedoch nicht auf diese Ausführungsformen beschränkt.

### Beispiele

In einen Rohrreaktor wurden 600 ml eines handelsüblichen Kupfer-Chromit-Katalysators in 3x3 mm großen Tabletten gegeben. Nach der Druckprobe mittels Stickstoff wurde zur Katalysatoraktivierung bei laufendem Stickstoffstrom die Temperatur langsam bis auf 180°C angehoben. Anschließend wurde dem laufenden Stickstoffstrom sukzessive Wasserstoff zugemischt bis auf eine Wasserstoffmenge von 60 Litern pro Stunde bei gleichbleibendem Stickstoffstrom. Dann wurde der Katalysator unter diesen Bedingungen über einen Zeitraum von 5 Stunden aktiviert.

Nach der Katalysatoraktivierung wurden im Rohrreaktor ein Wasserstoffdruck und eine Reaktionstemperatur gemäß den Bedingungen der Tabellen 1 und 2 eingestellt. Bei dem jeweils eingestellten Abgasstrom wurde die entsprechende Menge eines Hochsiedergemischs aus der Herstellung von Neopentylglykol kontinuierlich in den Rohrreaktor zugegeben und der abgeführte Abgasstrom in einen Hochdruckabscheider geleitet und über eine Standregelung in eine drucklose-Vorlage abgeführt. Die Spaltprodukte wurden mit rohem Neopentylglykol vermischt und die Mischung zu gereinigten Neopentylglykol aufdestilliert.

### Beispiele bei unterschiedlichen Temperaturen und Drücken:

Für die hydrierenden Spaltversuche kam Hochsiedermaterial aus der Neopentylglykolherstellung mit folgender Zusammensetzung zum Einsatz (gaschromatographische Analyse, Angaben in Prozent):

| | |
|---|---|
| Vorlauf | 0,6 |
| HPA | 18,3 |
| NPG | 20,8 |
| NPG-mono-formiat | 0,2 |
| NPG-di-formiat | 0,2 |
| Zwischenlauf | 0,1 |
| NPG-mono-isobutyrat | 4,5 |
| Cyclisches Acetal {HPA/NPG} | 0,2 |
| HPA-Tischtschenkoester/Di-isobuttersäure-NPG-Ester (TE, di-i-BNPG-Ester) | 46,6 |
| Nachlauf | 8,5 |

In den nachfolgenden Tabellen sind die Ergebnisse der Spaltversuche bei unterschiedlichen Temperaturen und Drücken zusammengefasst.

**Tabelle 1:**

| Hydrierende Spaltung der Hochsieder aus der Neopentylglykol-Herstellung (NPG-Einsatz) bei unterschiedlichen Temperaturen | | | | | |
|---|---|---|---|---|---|
| Druck | [MPa] | 8,0 | 8,0 | 8,0 | 8,0 |
| Kat Temp | [°C] | 180 | 185 | 190 | 200 |
| NPG Einsatz | [g/h] | 324 | 299 | 323 | 336 |
| Abgas | [l/h] | 260 | 255 | 220 | 230 |

| GC-Analysen | | | | | |
|---|---|---|---|---|---|
| Vorlauf | % | 2,4 | 3,5 | 3,1 | 8,4 |
| i-Butanol | % | 4,4 | 6,3 | 12,8 | 18,9 |
| NPG | % | 83,8 | 81,8 | 79,7 | 68,5 |
| NPG-mono-i-butyrat | % | 1,0 | 0,8 | 0,6 | 0,6 |
| TE+ di-i-BNPG-Ester | % | 6,0 | 5,5 | 1,8 | 2,3 |
| Summe Hochsieder | % | 2,4 | 2,1 | 2,0 | 1,3 |

Wie die Variation der Temperatur zeigt, nimmt mit steigender Spalttemperatur der NPG-Gehalt im hydrierten Produkt ab, gleichzeitig steigt jedoch der Anteil an iso-Butanol und Vorlaufkomponenten. Dieser Gang der Analysendaten deutet auf eine verstärkt einsetzende unselektive Spaltung von Neopentylglykol sowie der Hydroxypivalinaldehyd-Tischtschenkoester/Di-isobuttersäure-NPG-Ester, unter anderem zu iso-Butanol und Vorlaufkomponenten, bei einer Temperatursteigerung hin.

**Tabelle 2:**

| Hydrierende Spaltung der Hochsieder aus der Neopentylglykol-Herstellung (NPG-Einsatz) bei unterschiedlichen Drücken. | | | | | | | | |
|---|---|---|---|---|---|---|---|---|
| | | | | | | | | Vergleich |
| Druck | [MPa] | 20,0 | 18,0 | 16,0 | 14,0 | 12,0 | 8,0 | 6,0 |
| Kat Temp | [°C] | 180 | 180 | 180 | 180 | 180 | 180 | 180 |
| NPG Einsatz | [g/h] | 230 | 300 | 310 | 310 | 310 | 240 | 235 |
| Abgas | [l/h] | 260 | 270 | 280 | 230 | 230 | 250 | 280 |

| GC-Analysen | | | | | | | | |
|---|---|---|---|---|---|---|---|---|
| Vorlauf | % | 2,9 | 3,6 | 2,7 | 3,0 | 3,1 | 3,6 | 3,5 |
| i-Butanol | % | 4,2 | 4,1 | 4,8 | 4,4 | 3,9 | 5,6 | 6,2 |
| NPG | % | 91,9 | 90,0 | 88,8 | 87,8 | 85,7 | 83,7 | 78,2 |
| NPG-mono-i-butyrat | % | 0,1 | 0,2 | 0,3 | 0,4 | 0,6 | 0,7 | 1,3 |
| TE+ di-i-BNPG-Ester | % | 0,4 | 1,6 | 2,1 | 2,8 | 5,0 | 4,5 | 8,1 |
| Summe Hochsieder | % | 0,5 | 0,5 | 1,3 | 1,6 | 1,7 | 1,9 | 2,7 |

Aus dem Gang der Analysendaten zeigt sich die Druckabhängigkeit der Spaltrate des Hydrierkatalysators. Bei einem Druck von 20 MPa erzielt man eine nahezu vollständige Spaltung und Hydrierung der Tischtschenkoester und der Di-isobuttersäure-NPG-Ester zu Neopentylglykol.

### Hydrierversuch mit anschließender Destillation:

Die hydrierende Spaltung des Hochsieders aus der Neopentylglykolherstellung mit der zuvor genannten Zusammensetzung wurde unter den gleichen apparativen Bedingungen wie bei den bisherigen Versuchen durchgeführt. Bei einem Druck von 8 MPa und einer Temperatur von 180°C mit - einer Einsatzmenge von 300 g/h Hochsieder, entsprechend einer Katalysatorbelastung von VNh 0,5 h⁻¹, erfolgte die hydrierende Spaltung. Die Spaltprodukte wiesen folgende gaschromatographisch ermittelte Zusammensetzung (in Prozent) auf:

| | |
|---|---|
| Vorlauf (enthaltend Methanol, iso-Butanol) | 7,4 |
| HPA | 0,0 |
| NPG | 82,0 |
| NPG-mono-formiat | 0,0 |
| NPG-di-formiat | 0,0 |
| Zwischenlauf | 0,3 |
| NPG-mono-isobutyrat | 0,9 |
| Cyclisches Acetal {HPA/NPG} | 0,3 |
| H PA-Tischtschenkoester/Di-isobuttersäure-NPG-Ester | 6,2 |
| Nachlauf | 2,9 |

Unter diesen Bedingungen verlief die Spaltung mit einem Umsatz von 91 %, bezogen auf die Einsatzmenge an Hochsiedern, bei einer Selektivität zu Neopentylglykol von 97,1 %, entsprechend einer Ausbeute an Neopentylglykol von 89,0 %.

Das erhaltene Spaltprodukt wurde mit rohem Neopentylglykol im Verhältnis von 1 zu 10 Gewichtsteilen vermischt und die Mischung anschließend zum gereinigten Neopentylglykol destillativ an einer 40 Bödenkolonne bei Normaldruck und bei einem Rücklaufverhältnis von 1:1 aufarbeitet. Nach Abnahme der Vorfraktion bei einer Kopftemperatur im Bereich von 87 bis 100°C und bei einer Sumpftemperatur im Bereich von 100 bis 125°C erhielt man gereinigtes Neopentylglykol bei einer Kopftemperatur von 210°C und bei einer Sumpftemperatur im Bereich von 210 bis 260°C. Einsatzmischung, gereinigtes Neopentylglykol und Destillationsrückstand wiesen folgende gaschromatographisch ermittelte Zusammensetzung (in Prozent) auf:

| | Einsatzmischung | NPG-Fraktion | Rückstand |
|---|---|---|---|
| Vorlauf (enthaltend Methanol, iso-Butanol) | 19,7 | 0,1 | 0,0 |
| HPA | 0,1 | 0,0 | 0,1 |
| NPG | 72,2 | 99,6 | 83,6 |
| Tri-n-Propylamin | 6,0 | 0 | 0 |
| NPG-mono-formiat | 0,0 | 0,0 | 0,0 |
| NPG-di-formiat | 0,0 | 0,0 | 0,0 |
| Zwischenlauf | 0,1 | 0,0 | 0,4 |
| NPG-mono-isobutyrat | 0,5 | 0,2 | 3,8 |
| Cyclisches Acetal (HPA/NPG) | 0,1 | 0,1 | 0,4 |
| HPA-Tischtschenkoester/Di-isobuttersäure-NPG-Ester | 0,9 | 0,0 | 10,9 |
| Nachlauf | 0,4 | 0,0 | 0,8 |

## Patentansprüche

1. Verfahren zur Gewinnung von Neopentylglykol aus den bei der Umsetzung von Formaldehyd mit iso-Butyraldehyd anfallenden Hochsiedern, **dadurch gekennzeichnet, dass** die Hochsieder aus dem Verfahren zur Herstellung von Neopentylglykol abgetrennt werden und in einem separaten Hydrierreaktor lösungsmittelfrei in Gegenwart eines Kupfer-Chromit-Katalysators bei einer Temperatur von 140 bis 200°C und bei einem Druck von 7 bis 28 MPa mit Wasserstoff in der flüssigen Phase behandelt werden und die erhaltenen Spaltprodukte destillativ aufgearbeitet werden.

2. Verfahren nach Anspruch 1, **dadurch gekennzeichnet, dass** die Behandlung mit Wasserstoff bei einer Temperatur von 160 bis 200°C und bei einem Druck von 7 bis 20 MPa erfolgt.

3. Verfahren gemäß Anspruch 1 oder 2, **dadurch gekennzeichnet, dass** der Kupfer-Chromit-Katalysator als Aktivator zusätzlich Barium, Magnesium oder Mangan oder Gemische davon enthält.

4. Verfahren gemäß einem oder mehreren der Ansprüche 1 bis 3, **dadurch gekennzeichnet, dass** die erhaltenen Spaltprodukte mit rohem Neopentylglykol vermischt und die Mischung zu gereinigtem Neopentylglykol destillativ aufgearbeitet wird.

## Claims

1. Process for obtaining neopentyl glycol from the high boilers formed in the reaction of formaldehyde with isobutyraldehyde, **characterized in that** the high boilers are separated off from the process for preparing neopentyl glycol and treated in the liquid phase with hydrogen in the absence of solvent and in the presence of a copper-chromite catalyst at a temperature of from 140 to 200°C and a pressure of from 7 to 28 MPa in a separate hydrogenation reactor and the cracking products obtained are worked up by distillation.

2. Process according to Claim 1, **characterized in that** the treatment with hydrogen is carried out at a temperature of from 160 to 200°C and a pressure of from 7 to 20 MPa.

3. Process according to Claim 1 or 2, **characterized in that** the copper-chromite catalyst further comprises barium, magnesium or manganese or a mixture thereof as activator.

4. Process according to one or more of Claims 1 to 3, **characterized in that** the cracking products obtained are mixed with crude neopentyl glycol and the mixture is worked up by distillation to give purified neopentyl glycol.

## Revendications

1. Procédé pour l'obtention de néopentylglycol à partir des composés à haut point d'ébullition apparaissant dans la réaction du formaldéhyde avec de l'isobutyraldéhyde, **caractérisé en ce qu'**on sépare les composés à haut point d'ébullition provenant du procédé pour la production de néopentylglycol et dans un réacteur d'hydrogénation distinct on les traite par de l'hydrogène dans la phase liquide, sans solvant, en présence d'un catalyseur au chromite de cuivre à une température de 140 à 200 °C et sous une pression de 7 à 28 MPa et on soumet les produits de coupure obtenus à un traitement final par distillation.

2. Procédé selon la revendication 1, **caractérisé en ce que** le traitement par de l'hydrogène s'effectue à une température de 160 à 200 °C et sous une pression de 7 à 20 MPa.

3. Procédé selon la revendication 1 ou 2, **caractérisé en ce que** le catalyseur au chromite de cuivre contient comme activateur en outre du baryum, du magnésium ou du manganèse ou des mélanges de ceux-ci.

4. Procédé selon une ou plusieurs des revendications 1 à 3, **caractérisé en ce qu'**on mélange les produits de coupure obtenus avec du néopentylglycol brut et on soumet le mélange à un traitement final par distillation pour obtenir du néopentylglycol purifié.
